# EUROPEAN PATENT APPLICATION

(11) **EP 4 717 265 A1**
(43) Date of publication of application: **01.04.2026**
(21) Application number: 24741639.9
(22) Date of filing: 05.01.2024
(51) Int. Cl.: A61K 31/353, A61P 19/02

(54) **PHARMACEUTICAL COMPOSITION FOR PREVENTING OR TREATING DEGENERATIVE OSTEOARTHRITIS**

(30) Priority: 12.01.2023 KR 20230004453
(71) Applicant: Glaceum, Inc., Gyeonggi-do 16675 (KR)
(72) Inventor: YOO, Sang Ku, Suwon-si, Gyeonggi-do 16223 (KR); PARK, Hyung Soon, Seoul 06560 (KR); KIM, Tae Won, Gwangju-si, Gyeonggi-do 12789 (KR); LEE, Hyeong Min, Suwon-si, Gyeonggi-do 16705 (KR); JEON, Hyun Ju, Hwaseong-si, Gyeonggi-do 18475 (KR); CHOI, Leo Sungwong, Yongin-si, Gyeonggi-do 16918 (KR)
(74) Representative: Ipsilon
(86) International application number: PCT/KR2024/000267
(87) International publication number: WO 2024/151017

(57) **Abstract**

The present invention relates to a pharmaceutical composition for preventing or treating degenerative arthritis, containing a pyrano[2,3-f]chromene derivative compound, a pharmaceutically acceptable salt thereof, or a solvate thereof.

## Description

### Technical Field

The present invention relates to a pharmaceutical composition for preventing or treating degenerative arthritis.

### Background Art

Osteoarthritis is a disease in which the articular cartilage covering the joint surface of the bone wears out, exposing the subchondral bone, and the synovial membrane surrounding the joint becomes inflamed, causing pain and deformity. It is also commonly called degenerative arthritis and is the most common joint disease. Osteoarthritis is caused by damage to the soft cartilage that acts as a cushion between the bones for certain reasons. These reasons include genetic factors, obesity, joint trauma, and cartilage damage caused by inflammation. If a person suffers from joint disease for a long time from childhood, osteoarthritis may occur, and in this case, degenerative arthritis may occur even at a relatively young age. If osteoarthritis occurs in the spine, back pain occurs. In severe cases, tingling nerve symptoms may occur, which may be mistaken for a herniated lumbar disc. If osteoarthritis occurs in the hip joint, pain may occur or the range of motion of the joint may be restricted, making walking awkward. Osteoarthritis also frequently occurs in the finger joints, especially in middle-aged women. When osteoarthritis occurs in the fingers, the finger joints gradually thicken and become painful.

Treatment methods for osteoarthritis broadly include drug therapy, physical therapy, lifestyle modification, and surgical treatment. Most drugs serve to reduce pain and swelling and slow the progression of the disease, but they are not fundamental therapeutic agents. The most commonly used drugs are nonsteroidal anti-inflammatory drugs (NSAIDs) and steroid-based injections. Nonsteroidal anti-inflammatory drugs have excellent anti-inflammatory and analgesic effects, but if they are taken for a long period of time, there is a possibility of side effects, including gastrointestinal diseases, or cardiovascular diseases such as heart attack, stroke, and myocardial infarction. In addition, steroid-based injections have temporary effects, and if they are administered frequently, there is a risk of damaging cartilage.

The pain that patients with osteoarthritis feel is nociceptive pain, which is a signal that the protection and recovery of cartilage tissue from damage is needed. Nociceptive pain is characterized in that the pain disappears immediately when the threatening stimulus disappears or the tissue recovers. However, in patients with osteoarthritis, the pain persists even when the external stimulus disappears. Osteoarthritis is an endogenous disease that originates from the human body itself, not an external stimulus. Degenerative diseases are closely related to aging, and the decline in cellular metabolism with aging leads to cell death. Mitochondrial dysfunction and metabolic decline in the chondrocytes of osteoarthritis patients gradually induce cell death, and our body detects the same as a danger signal and causes persistent pain. Therefore, improving the metabolism of chondrocytes and inhibiting cell death is a new osteoarthritis treatment method that not only suppresses pain but also fundamentally addresses the intrinsic cause.

### DISCLOSURE

### Technical Problem

An object of the present invention is to provide a pharmaceutical composition for preventing or treating degenerative arthritis.

However, the objects of the present invention are not limited to the object mentioned above, and other objects not mentioned may be clearly understood by those skilled in the art from the following description.

### Technical Solution

One embodiment of the present invention provides a pharmaceutical composition for preventing or treating degenerative osteoarthritis, containing a pyrano[2,3-f]chromene derivative compound of Formula 1 below, a pharmaceutically acceptable salt thereof, or a solvate thereof:

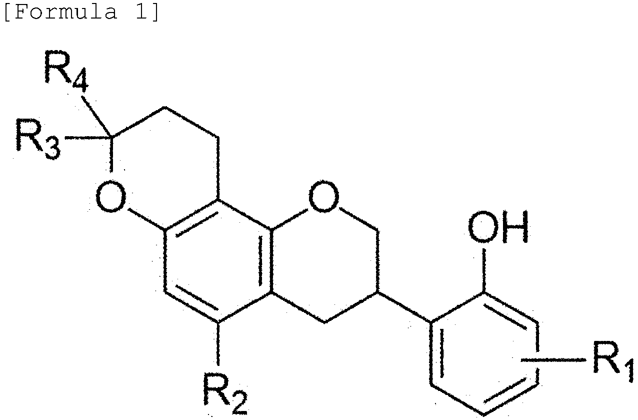

In Formula 1 above, R₁ is a hydrogen atom, a substituted or unsubstituted linear or branched C₁-C₆ alkyl group, a halogen atom, a substituted or unsubstituted linear or branched C₁-C₆ alkoxy group, or a substituted or unsubstituted linear or branched C₁-C₄ thioalkyl group; R₂ is a hydrogen atom, methyl, ethyl, methoxy or ethoxy; and R₃ and R₄ are each independently a hydrogen atom or a C₁-C₂ alkyl group, wherein substituents of the substituted alkyl, the substituted alkoxy and the substituted thioalkyl are each independently a linear or branched C₁-C₅ alkyl group, a halogen atom, a linear or branched C₁-C₅ alkoxy group, or a linear or branched C₁-C₃ thioalkyl group.

### Advantageous Effects

The pharmaceutical composition containing a pyrano[2,3-f]chromene derivative compound, a pharmaceutically acceptable salt thereof, or a solvate thereof according to one embodiment of the present invention has excellent preventive and therapeutic effects against degenerative arthritis.

The effects of the present invention are not limited to the effects mentioned above, and effects not mentioned may be clearly understood by those skilled in the art from the present specification and the accompanying drawings.

### Brief Description of Drawings

FIG. 1 shows the results of measuring pain levels in a MIA-OA rat model according to Experimental Example 1.
FIG. 2 shows the results of measuring weight bearing in a MIA-OA rat model according to Experimental Example 2.
FIG. 3 shows the results of histopathology of the knee joint tissue of a MIA-OA rat model according to Experimental Example 3.
FIG. 4 shows the results of measuring OARSI and Mankin scores through the results of histopathology according to Experimental Example 3.
FIG. 5 shows the results of quantifying IL-6 changes in knee joint tissue through immunohistochemical analysis according to Experimental Example 4.
FIG. 6 shows the results of quantifying MCP-1 changes in knee joint tissue through immunohistochemical analysis according to Experimental Example 4.
FIG. 7 shows the results of quantifying MMP3 changes in knee joint tissue through immunohistochemical analysis according to Experimental Example 4.
FIG. 8 shows the results of quantifying RIPK3 changes in knee joint tissue through immunohistochemical analysis according to Experimental Example 4.

### Best Mode

All technical terms used in the present specification, unless otherwise defined, have the same meaning as commonly understood by those skilled in the art to which the present invention pertains. In addition, although preferred methods or samples are described in the present specification, those similar or equivalent thereto are also included in the scope of the present invention.

One embodiment of the present invention provides a pharmaceutical composition for preventing or treating degenerative osteoarthritis, containing a pyrano[2,3-f]chromene derivative compound of Formula 1 below, a pharmaceutically acceptable salt thereof, or a solvate thereof:

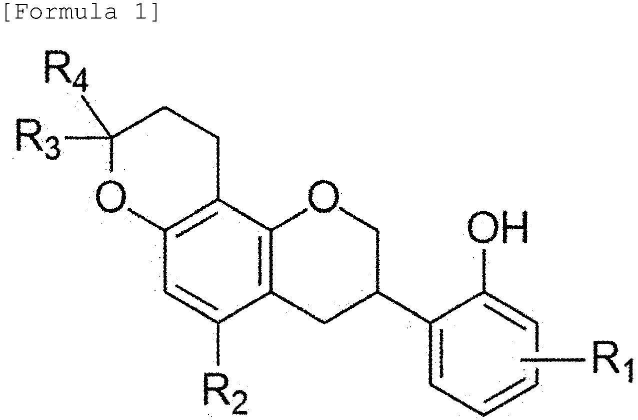

In Formula 1 above, R₁ is a hydrogen atom, a substituted or unsubstituted linear or branched C₁-C₆ alkyl group, a halogen atom, a substituted or unsubstituted linear or branched C₁-C₆ alkoxy group, or a substituted or unsubstituted linear or branched C₁-C₄ thioalkyl group; R₂ is a hydrogen atom, methyl, ethyl, methoxy or ethoxy; and R₃ and R₄ are each independently a hydrogen atom or a C₁-C₂ alkyl group, wherein substituents of the substituted alkyl, the substituted alkoxy and the substituted thioalkyl are each independently a linear or branched C₁-C₅ alkyl group, a halogen atom, a linear or branched C₁-C₅ alkoxy group, or a linear or branched C₁-C₃ thioalkyl group.

The pharmaceutical composition containing a pyrano[2,3-f]chromene derivative compound, a pharmaceutically acceptable salt thereof, or a solvate thereof according to one embodiment of the present invention has excellent preventive and therapeutic effects against degenerative osteoarthritis, particularly primary osteoarthritis.

In one embodiment of the present invention, the degenerative osteoarthritis may include osteoporosis, knee arthritis, and/or knee pain. In addition, the degenerative osteoarthritis may be primary osteoarthritis or secondary osteoarthritis. The primary osteoarthritis may be idiopathic osteoarthritis and osteoarthritis caused by aging, obesity, injury, etc. The secondary osteoarthritis may be chondromalacia patella or patellofemoral degenerative arthritis secondary to primary osteoarthritis.

The pharmaceutical composition containing a pyrano[2,3-f]chromene derivative compound, a pharmaceutically acceptable salt thereof, or a solvate thereof according to the present invention has excellent preventive and therapeutic effects against idiopathic osteoarthritis and secondary osteoarthritis such as chondromalacia patella or patellofemoral degenerative arthritis secondary to primary osteoarthritis.

In the present invention, the degenerative osteoarthritis is a disease in which inflammation and pain occur due to damage to joint bones and ligaments caused by gradual damage or degenerative changes in the cartilage protecting the joint.

According to one embodiment of the present invention, R₁ in Formula 1 above may be a substituted or unsubstituted linear or branched C₁-C₆ alkoxy group. Specifically, R₁ may be an unsubstituted linear or branched C₁-C₆ alkoxy group. In addition, R₂ in Formula 1 may be a hydrogen atom, and R₃ and R₄ may each independently be a C₁-C₂ alkyl group. When R₁ to R₄ in Formula 1 are as described above, the pharmaceutical composition containing the pyrano[2,3-f]chromene derivative compound of Formula 1, a pharmaceutically acceptable salt thereof, or a solvate thereof is chemically stable and has excellent preventive and therapeutic effects against degenerative osteoarthritis, particularly primary osteoarthritis.

According to one embodiment of the present invention, the compound represented by Formula 1 may comprise any one of the following compounds:

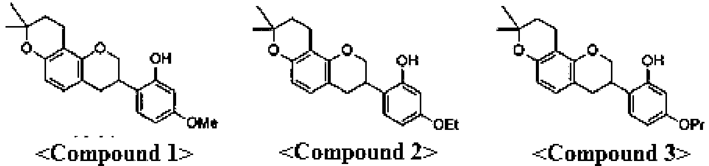

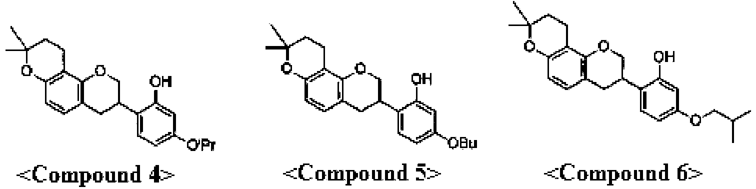

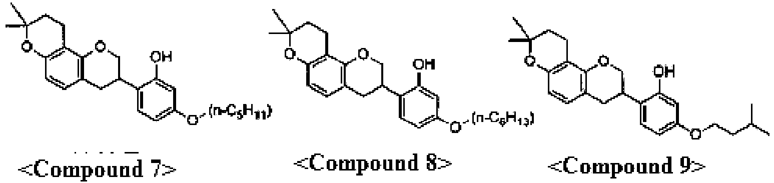

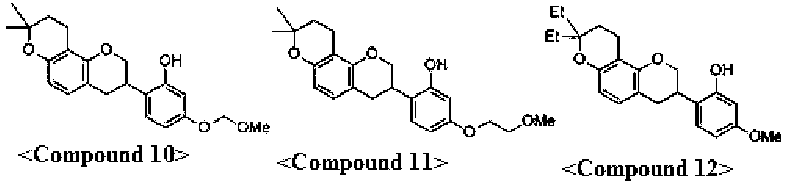

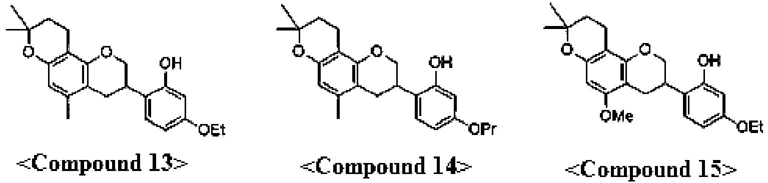

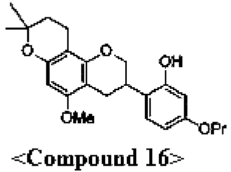

The pharmaceutical composition containing any one of Compounds 1 to 16 above, a pharmaceutically acceptable salt thereof, or a solvate thereof is chemically stable and has excellent preventive and therapeutic effects against degenerative osteoarthritis, particularly primary osteoarthritis.

In addition, the pharmaceutical composition may have an excellent preventive or therapeutic effect against degenerative osteoarthritis even when it is administered once a day. In addition, the pharmaceutical composition may exhibit an excellent anti-degenerative osteoarthritis effect even when the active ingredient is administered at a relatively low dose. In particular, the pharmaceutical composition containing any one of Compounds 1 to 16 above may have a better anti-degenerative osteoarthritis effect than the pharmaceutical composition containing any one of Compounds 17 to 28 described below.

According to one embodiment of the present invention, the compound represented by Formula 1 may comprise any one of the following compounds:

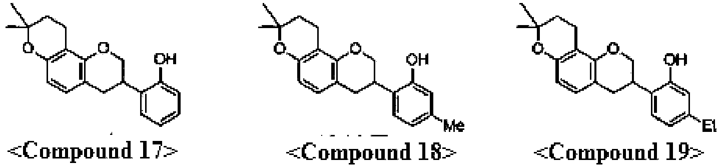

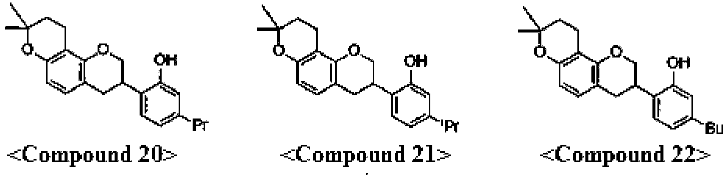

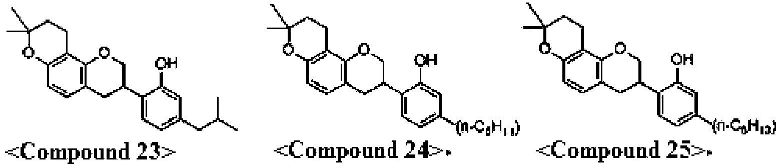

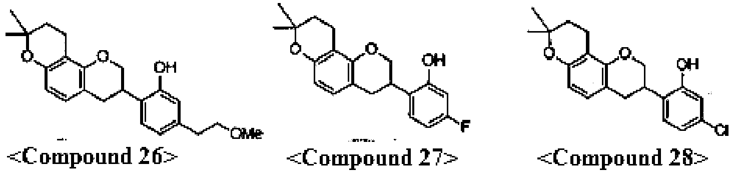

The pharmaceutical composition containing any one of Compounds 17 to 28, a pharmaceutically acceptable salt thereof, or a solvate thereof may exhibit an excellent effect against degenerative osteoarthritis.

The pharmaceutical composition containing the pyrano[2,3-f]chromene derivative compound of Formula 1, a pharmaceutically acceptable salt thereof, or a solvate thereof has excellent preventive and therapeutic effects against degenerative osteoarthritis, particularly primary osteoarthritis.

According to one embodiment of the present invention, the pyrano[2,3-f]chromene derivative compound of Formula 1 may induce an anti-inflammatory response in joint cartilage tissue by inhibiting chemokines and cytokines that induce an inflammatory response, such as IL-6 and MCP-1, in knee joint cartilage tissue, and inhibiting MMP3, a cartilage-degrading protein, and RIPK3, which is involved in chondrocyte apoptosis. Therefore, the pyrano[2,3-f]chromene derivative compound may have an excellent effect on the prevention or treatment of degenerative osteoarthritis, especially primary osteoarthritis.

According to one embodiment of the present invention, the pharmaceutically acceptable salt of the compound of Formula 1 may mean that the compound of Formula 1 forms a salt with a free acid, which is present as an acid addition salt. The compound of Formula 1 may form a pharmaceutically acceptable acid addition salt according to a conventional method known in the art. As the free acid, an organic acid or an inorganic acid may be used. The inorganic acid may be hydrochloric acid, hydrobromic acid, sulfuric acid, phosphoric acid, or the like, and the organic acid may be citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, galacturonic acid, embonic acid, glutamic acid, aspartic acid, or the like.

The pharmaceutical composition according to one embodiment of the present invention may contain, in addition to the pyrano[2,3-f]chromene derivative compound of Formula 1, a pharmaceutically acceptable salt thereof, or a solvate thereof, a pharmaceutically acceptable carrier, excipient or diluent. Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In one embodiment of the present invention, the pyrano[2,3-f]chromene derivative compound of Formula 1 may be produced according to the same method as described in Korean Patent Application Publication Nos. 10-2015-0075030 and 10-2018-0002539. However, the present invention is not limited thereto.

According to one embodiment of the present invention, the pharmaceutical composition may be formulated into conventional pharmaceutical formulations known in the art. The formulations may be prepared and administered in any dosage forms, including, but not limited to, oral administration formulations, injections, suppositories, transdermal administration formulations, and nasal administration formulations. Preferably, the composition may be formulated into oral administration formulations and injections.

Each of the formulations may be prepared by adding a pharmaceutically acceptable carrier necessary for the preparation of each formulation. In the present specification, the term "pharmaceutically acceptable carrier" is used to refer to any component other than a pharmaceutically active ingredient. "Pharmaceutically acceptable" means a property that does not cause any pharmaceutically undesirable change via interaction between different components present in the composition (for example, via interaction between carriers or via interaction between the pharmaceutically active ingredient and a carrier). Selection of the pharmaceutically acceptable carrier may vary depending on factors such as the properties of a particular dosage form, the mode of administration, and the effects of the carrier on solubility and stability.

According to one embodiment of the present invention, the pharmaceutically acceptable carrier contained in the pharmaceutical composition for oral administration may be at least one selected from among a diluent, a binder, a disintegrant, a glidant (or lubricant), an adsorbent, a stabilizer, a solubilizing agent, a sweetener, a coloring agent, and a flavoring agent, without being limited thereto.

The diluent refers to any excipient that is added to increase the volume of the composition to formulate the composition into a dosage form with an appropriate size. The diluent may be at least one selected from among starch (e.g., potato starch, corn starch, wheat starch, and pregelatinized starch), microcrystalline cellulose (e.g., low-hydrated microcrystalline cellulose), lactose (e.g., lactose monohydrate, anhydrous lactose, and spray lactose), glucose, sorbitol, mannitol, sucrose, alginate, alkaline earth metal salts, clay, polyethylene glycol dicalcium phosphate, anhydrous calcium hydrogen phosphate, and silicon dioxide, without being limited thereto. In the present invention, the diluent may be used in an amount ranging from 5 wt% to 50 wt% based on the total amount of the pharmaceutical composition. For example, the diluent may be used in an amount ranging from 10 wt% to 35 wt% based on the total amount of the composition for tabletting and quality maintenance.

The binder refers to a material that is used to impart adhesiveness to powdery materials to facilitate compression of the materials, and improve fluidity. The binder may be at least one selected from among starch, microcrystalline cellulose, highly dispersible silica, mannitol, lactose, polyethylene glycol, polyvinylpyrrolidone, cellulose derivatives (e.g., hydroxypropyl methylcellulose, hydroxypropyl cellulose, or low-substituted hydroxypropyl cellulose), natural gum, synthetic gum, povidone, co-povidone, and gelatin, without being limited thereto. In the present invention, the binder may be used in an amount of 2 wt% to 15 wt% based on the total weight of the pharmaceutical composition. For example, the binder may be used in an amount of 1 wt% to 3 wt% based on the total weight of the pharmaceutical composition for tabletting and quality maintenance.

The disintegrant refers to a material that is added to facilitate collapse or disintegration of a solid dosage form after administrated *in vivo.* Examples of the disintegrant include, but are not limited to, starch or modified starch, such as sodium starch glycolate, corn starch, potato starch, or pregelatinized starch, clay, such as bentonite, montmorillonite, or veegum, cellulose, such as microcrystalline cellulose, hydroxypropyl cellulose, or carboxymethyl cellulose, algins, such as sodium alginate or alginic acid, a cross-linked cellulose, such as croscarmellose sodium, gum such as guar gum or xanthan gum, a cross-linked polymer such as cross-linked polyvinylpyrrolidone (crospovidone), and an effervescent agent such as sodium bicarbonate or citric acid, which may be alone or in combination. In the present invention, the disintegrant may be used in an amount ranging from 2 wt% to 15 wt% based on the total weight of the pharmaceutical composition. For example, the disintegrant may be used in an amount of 4 wt% to 10 wt% based on the total weight of the pharmaceutical composition for tabletting and quality maintenance.

The glidant or lubricant refers to a material that prevents the adhesion of powder to a compressing system and improves the flowability of granules. Examples of the glidant include, but are not limited to, light anhydrous silicic acid, talc, stearic acid, a metal salt (magnesium salt, calcium salt, or the like) of stearic acid, sodium lauryl sulfate, hydrogenated vegetable oil, sodium benzoate, sodium stearyl fumarate, glycerylbehenate, glycerylmonostearate, and polyethylene glycol, which may be used alone or in combination.

In the present invention, the glidant may be used in an amount of 0.1 wt% to 5 wt% based on the total weight of the pharmaceutical composition. For example, the glidant may be used in an amount of 1 wt% to 3 wt% based on the total weight of the pharmaceutical composition for tabletting and quality maintenance.

Examples of the adsorbent include, but are not limited to hydrated silicon dioxide, light anhydrous silicic acid, colloidal silicon dioxide, magnesium aluminometasilicate, microcrystalline cellulose, lactose, and cross-linked polyvinylpyrrolidone, which may be used alone or in combination.

The stabilizer may be at least one selected from antioxidants, such as butylhydroxyanisol, butylhydroxytoluene, carotene, retinol, ascorbic acid, tocopherol, tocopherolpolyethylene glycol succinic acid, or propyl gallate; cyclic sugar compounds such as cyclodextrin, carboxyethyl cyclodextrin, hydroxypropyl cyclodextrin, sulfobutylether, or cyclodextrin; and organic acids such as phosphoric acid, lactic acid, acetic acid, citric acid, tartaric acid, succinic acid, maleic acid, fumaric acid, glycolic acid, propionic acid, gluconic acid, or glucuronic acid, without being limited thereto.

Optionally, the pharmaceutical composition may contain known additives to enhance palatability by enhancing the sense of taste. For example, a sweetener such as sucralose, sucrose, fructose, erythritol, acesulfame potassium, sugar alcohol, honey, sorbitol, or aspartame may be added to more effectively mask bitterness and maintain the stability and quality of the formulation. Further, an acidifier such as citric acid or sodium citrate; a natural flavoring agent such as Japanese apricot flavor, lemon flavor, pineapple flavor, or herbal flavor; or a natural dye such as natural fruit juice, chlorophyllin, or flavonoid may be used.

The pharmaceutical composition for oral administration may be a solid formulation, a semi-solid formulation or a liquid formulation for oral administration. Examples of the solid formulation for oral administration include, but are not limited to, a tablet, a pill, a hard or soft capsule, a powder, a fine granule, a granule, a powder for reconstitution into a solution or suspension, a lozenge, a wafer, an oral strip, a dragee, a chewable gum, and the like. Examples of the liquid formulation for oral administration include a solution, suspension, emulsion, syrup, elixir, spirit, aromatic waters, lemonade, extract, precipitate, tincture, and oily medicine. Examples of the semi-solid formulation include, but are not limited to, aerosol, cream, gel and the like. The pharmaceutical composition according to the present invention may be formulated into an injectable formulation, and when the pharmaceutical composition may be formulated into an injectable formulation, it may contain, as a diluent, a nontoxic buffer solution which is isotonic to blood, for example, phosphate-buffered saline with a pH of 7.4. The pharmaceutical composition may contain other diluents or additives in addition to the buffered saline.

The carrier used in the above-mentioned formulation and the formulation may be selected and prepared as widely known in the art, and may be prepared, for example, according to the method described in Remington's Pharmaceutical Science (latest edition).

The dose and administration time of the pharmaceutical composition according to the present invention may vary depending on the subject's age, sex, condition, body weight, the route of administration, the administration frequency, and the type of drug. The daily dose is about 0.1 mg/kg to about 300 mg/kg, preferably about 1 mg/kg to 200 mg/kg based on the active ingredient, that is, the compound of Formula 1. The dose may be appropriately increased and decreased according to the type of disease, the degree of progress of the disease, the route of administration, sex, age, body weight, and the like.

To obtain a desired effect, the pharmaceutical composition according to the present invention may be arbitrarily administered several times so that the total daily dose for an adult is 1 to 100 mg/kg, specifically 25 to 75 mg/kg, more specifically 50 mg/kg, based on the compound of Formula 1 as the active ingredient.

In one embodiment of the present invention, the pharmaceutical composition according to the present invention may reduce an inflammatory factor. Specifically, the pharmaceutical composition may reduce an inflammatory factor by 75 to 25%, more specifically 50%.

Another embodiment of the present invention provides a method for preventing or treating degenerative osteoarthritis, comprising a step of administering a composition containing a pyrano[2,3-f]chromene derivative compound of Formula 1 below, a pharmaceutically acceptable salt thereof, or a solvate thereof to a subject suspected of having degenerative osteoarthritis:

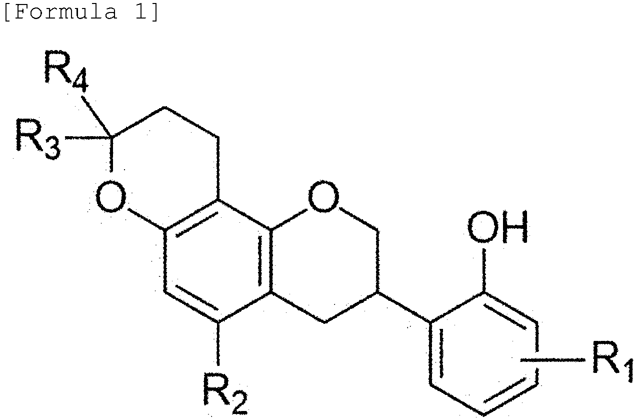

In Formula 1 above, R₁ is a hydrogen atom, a substituted or unsubstituted linear or branched C₁-C₆ alkyl group, a halogen atom, a substituted or unsubstituted linear or branched C₁-C₆ alkoxy group, or a substituted or unsubstituted linear or branched C₁-C₄ thioalkyl group; R₂ is a hydrogen atom, methyl, ethyl, methoxy or ethoxy; and R₃ and R₄ are each independently a hydrogen atom or a C₁-C₂ alkyl group, wherein substituents of the substituted alkyl, the substituted alkoxy and the substituted thioalkyl are each independently a linear or branched C₁-C₅ alkyl group, a halogen atom, a linear or branched C₁-C₅ alkoxy group, or a linear or branched C₁-C₃ thioalkyl group.

Still another embodiment of the present invention provides the use of a composition containing a pyrano[2,3-f]chromene derivative compound of Formula 1 below, a pharmaceutically acceptable salt thereof, or a solvate thereof, for the prevention or treatment of degenerative osteoarthritis.

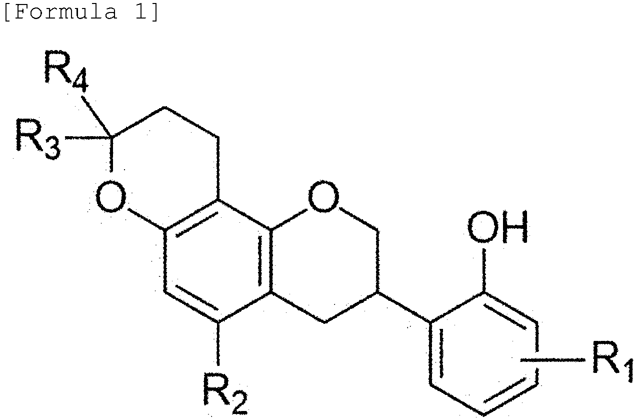

In Formula 1 above, R₁ is a hydrogen atom, a substituted or unsubstituted linear or branched C₁-C₆ alkyl group, a halogen atom, a substituted or unsubstituted linear or branched C₁-C₆ alkoxy group, or a substituted or unsubstituted linear or branched C₁-C₄ thioalkyl group; R₂ is a hydrogen atom, methyl, ethyl, methoxy or ethoxy; and R₃ and R₄ are each independently a hydrogen atom or a C₁-C₂ alkyl group, wherein substituents of the substituted alkyl, the substituted alkoxy and the substituted thioalkyl are each independently a linear or branched C₁-C₅ alkyl group, a halogen atom, a linear or branched C₁-C₅ alkoxy group, or a linear or branched C₁-C₃ thioalkyl group.

The pharmaceutical composition according to the present invention may contain the compound of Formula I according to the present invention in an amount of about 1 to 50 wt%, preferably 10 to 40 wt%, based on the total weight of the composition.

### Mode for Invention

Hereinafter, the present invention will be described in detail through examples in order to explain the present invention in detail. However, the examples according to the present invention may be modified into various different forms, and the scope of the present invention should not be construed as being limited to the examples described below. The examples in the present specification are provided to more completely explain the present invention to those skilled in in the art.

### Experimental Example 1: Evaluation of Pain Suppression Effect in MIA-Induced OA Rat Model

### Experimental Example 1-1. Experimental Preparation

All animals were allowed to acclimatize for one week after introduction to the laboratory environment. On the day of the start of the experiment, monosodium iodoacetate (MIA, Sigma, Cat. No. I2512) was dissolved in saline for injection at a concentration of 30 mg/mL, and 50 µL of the solution was administered once on the day of the start of the experiment. The animals were divided into groups as follows. The animals were randomly distributed so that the average of each group was distributed as evenly as possible according to the pain threshold evaluation values ranked, so as to obtain the number specified in the "Constitution of Test Groups" table. Individual identification was done by writing numbers directly on the tails of the animals. Individual identification cards of different colors were attached to the housing boxes according to each dose. An animal room usage record sheet containing the test number, animal room usage period, principal investigator name, subinvestigator name, emergency contact information, etc. was attached to the animal room entrance. The test groups consisted of WT, vehicle, 25 mg/kg Compound 2, and 50 mg/kg Compound 2, respectively, which were administered orally once a day for a total of 21 days starting from 3 days after MIA administration.

The nociceptive pain threshold (g) was measured using a dynamic plantar aesthesiometer (Ugo Basile, 37400, Comerio, Italy), a device in which the force applied to the animal's paw gradually increases over a certain period of time. Before measurement, the animal was placed in an acrylic box with a wire mesh bench at the bottom and allowed to acclimate for 5 minutes. The nociceptive pain threshold was measured by applying a gradually increasing force from 0 to 50 g over 10 seconds to the center of the right hind paw of each animal using a metal filament, and measuring the weight at which the animal exhibited a paw withdrawal behavior. To avoid tissue damage, the cut-off threshold was set at 50 g. The baseline value was obtained by measurement before arthritis induction (day 0), and then measurements were done before test substance administration (day 3) and at a set time twice a week after intra-articular test substance administration. The results for the nociceptive pain threshold were calculated as paw withdrawal latency (sec) and paw withdrawal threshold (g).

### Experimental Example 1-2. Experimental Results

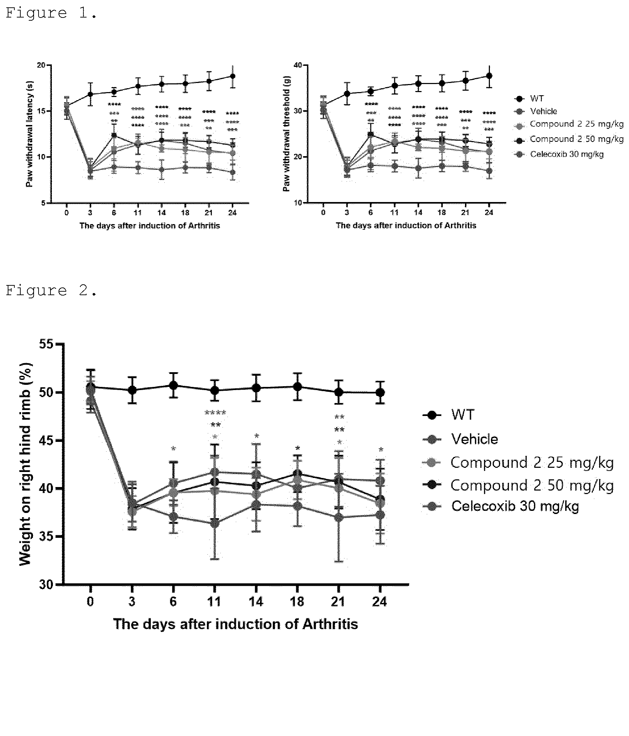

As a result of measuring pain caused by cartilage damage in the OA animal model according to Experimental Example 1-1, it could be confirmed that pain in the OA model was gradually reduced by administering Compound 2. It could be confirmed that Compound 2 exhibited efficacy equivalent to that of the positive control, celecoxib (FIG. 1).

### Experimental Example 2: Confirmation of Reduced Weight Bearing in MIA-Induced OA Rat Model

### Experimental Example 2-1. Preparation for Experiment on Degree of Reduction in Weight Bearing

Weight bearing was measured using the same model as that in Experimental Example 1. The weight-bearing measurement test is an experiment to measure the change in weight bearing (or weight distribution) between the normal hind limb (left) and the arthritis-induced hind limb (right), which is caused by pain in the hind limb knee with unilateral arthritis induced. The weight bearing of each hind paw was measured using an incapacitance meter (Model 600, IITC, USA), a device for measuring weight bearing. Since weight bearing can change depending on the posture in which the animal sets foot, the animal was correctly positioned in the holder so that both feet could be set symmetrically. Also, the person in charge was set to one person to minimize errors. Before measurement, the animal was placed in an acrylic box and allowed to acclimate for 5 minutes.

When each animal was correctly positioned in the holder, the machine was operated, the measurement time was set to 5 seconds, and a total of two measurements were taken and averaged to determine the weight bearing (g) of each paw. The baseline value was obtained by measurement before arthritis induction (day 0), and then measurements were done before test substance administration (day 3) and at a set time twice a week after intra-articular test substance administration. The results of the weight-bearing measurement test were converted into weight bearing ratio using the following calculation formula and analyzed.

### Experimental Example 2-2. Results of Experimental Example 2-1

As a result of measuring the weight bearing following cartilage damage in the OA animal model according to Experimental Example 2-1, it could be confirmed that the weight bearing in the OA model was reduced by administering Compound 2.

In addition, it could be confirmed that Compound 2 exhibited efficacy equivalent to that of celecoxib, a positive control (FIG. 2).

### Experimental Example 3: Histopathological Analysis After Treatment in MIA-OA Animal Model

### Experimental Example 3-1: Histopathological Analysis After Treatment in MIA-OA Animal Model

Histopathological analysis of knee joint tissue was performed using the same model as that in Experimental Example 1. For histopathological analysis, Safranin O staining was performed, and two evaluators scored the severity of osteoarthritis according to the following evaluation criteria. As the analysis criteria, OARSI and total Mankin scores were analyzed. The OARSI analysis criteria were scored as follows: 0: normal cartilage surface; 1: cartilage surface is normal but cell death is found; 2: disruption of the cartilage surface and fibrosis of the surface layer; 3: splitting along the longitudinal axis of cartilage; 4: damage and infiltration to the middle part of cartilage; 5: exposure of stromal bone area; and 6: complete cartilage damage and formation of osteophytes. The total Mankin score analysis criteria were analyzed for four items: structure (0-6), cell (0-3), Safranin O (0-4), and tidemark (0-1). A higher score for each item indicates more damage to chondrocytes cells and structure.

### Experimental Example 3-2. Experimental Results for Histopathological Analysis

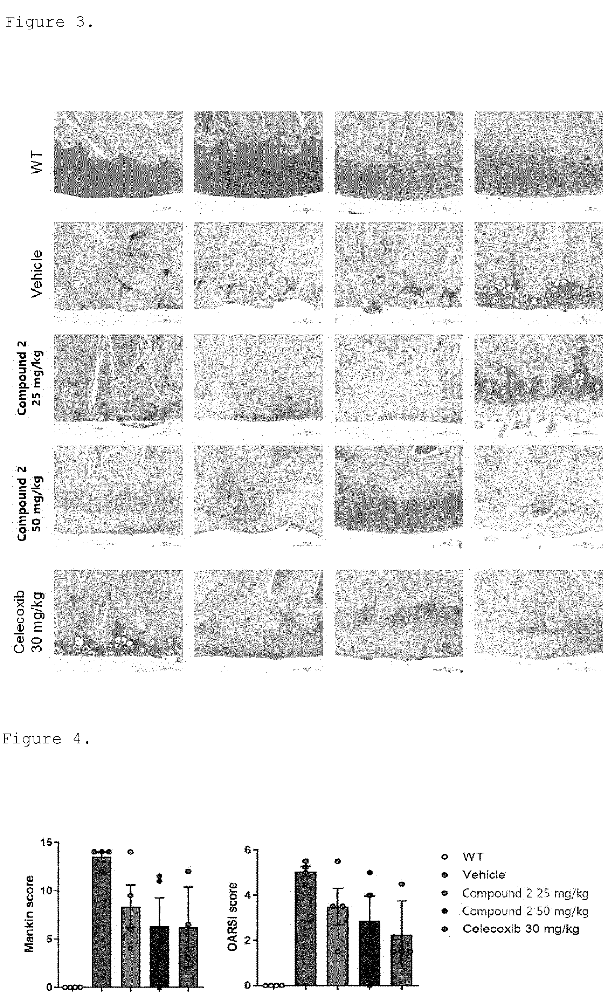

As a result of performing the histopathology of the knee joint in the OA animal model according to Experimental Example 3-1, Safranin 0 staining of the cartilage tissue in the OA model was improved by administering Compound 2 (FIG. 3).

In addition, the ORASI and Mankin scores, which indicate the severity of osteoarthritis, were reduced by administering Compound 2. It could be confirmed that Compound 2 exhibited efficacy equivalent to that of celecoxib, a positive control (FIG. 4) .

### Experimental Example 4: Immunohistochemical Analysis After Treatment in MIA-OA Animal Model

### Experimental Example 4-1. Immunohistochemical Analysis in MIA-OA Animal Model

The histopathological analysis of knee joint tissue was performed using the same model as that in Experimental Example 1. For immunochemical analysis of joint tissue, the joint tissue was stained with specific IL-6, MCP-1, MMP3, and RIPK3 antibodies, and imaged under a microscope at 400x magnification. Then, the Dab-positive area expressing each cytokine in the synovium was extracted using ImageJ software (Wayne Rasband, NIH, USA) and expressed as a percentage relative to the total nuclear area.

### Experimental Example 4-2. Results of Immunohistochemical Analysis After Treatment in MIA-OA Animal Model

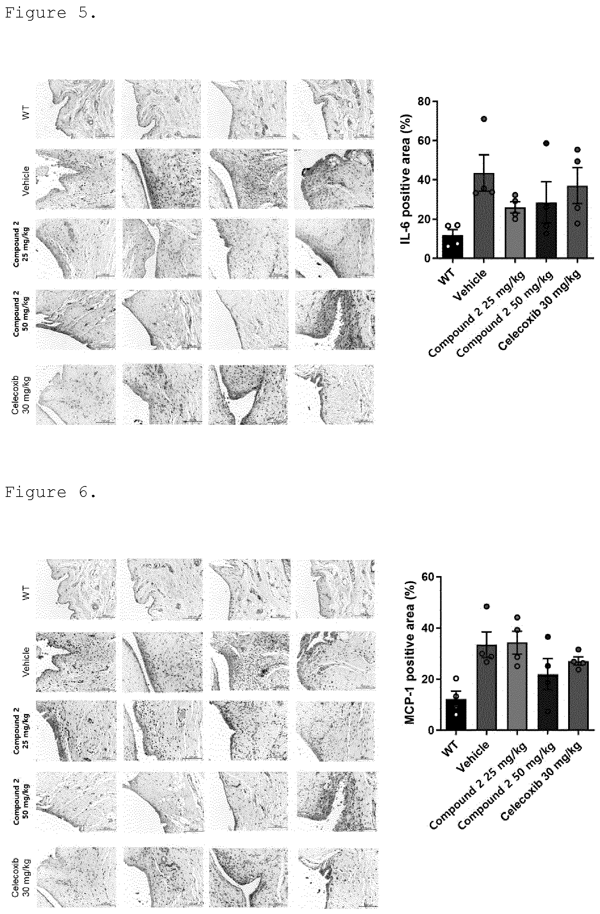

As a result of performing immunohistochemical analysis of the knee joint in the OA animal model according to Experimental Example 4-1, it could be confirmed that IL-6 and MCP-1, proteins that cause inflammation in the OA model, were inhibited by administering Compound 2 (FIGS. 5 and 6).

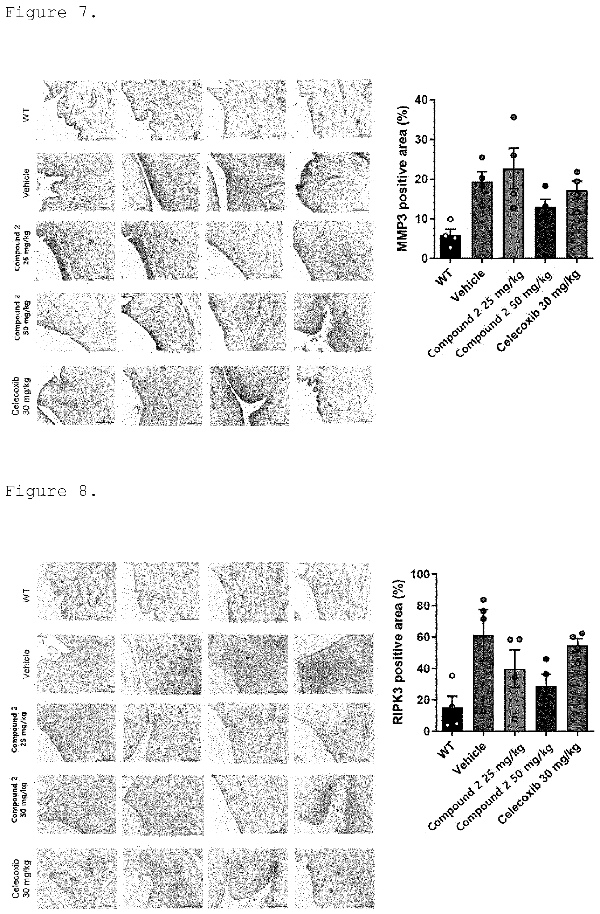

In addition, it could be confirmed that, when Compound 2 was administered, MMP3, a protein that degrades cartilage, was also reduced, and RIPK3, which is involved in chondrocyte apoptosis, was inhibited. At this time, it could be confirmed that Compound 2 exhibited better efficacy than celecoxib, a positive control (FIGS. 7 and 8).

So far, the present invention has been described with reference to the preferred embodiments. Those skilled in the art will appreciate that the present invention can be implemented in modified forms without departing from the essential features of the present invention. Therefore, the disclosed embodiments should be considered in descriptive sense only and not for purposes of limitation. Therefore, the scope of the present invention is defined not by the detailed description of the present invention but by the appended claims, and all differences within a range equivalent to the scope of the appended claims should be construed as being included in the present invention.

### Industrial Applicability

An object of the present invention is to provide a pharmaceutical composition for preventing or treating degenerative arthritis. Specifically, the present invention relates to a pharmaceutical composition for preventing or treating degenerative arthritis, containing a pyrano[2,3-f]chromene derivative compound, a pharmaceutically acceptable salt thereof, or a solvate thereof, and thus is industrially applicable in terms of the treatment, amelioration, alleviation, and prevention of degenerative arthritis.

## Claims

1. A pharmaceutical composition for preventing or treating degenerative osteoarthritis, comprising a pyrano[2,3-f]chromene derivative compound of Formula 1 below, a pharmaceutically acceptable salt thereof, or a solvate thereof: 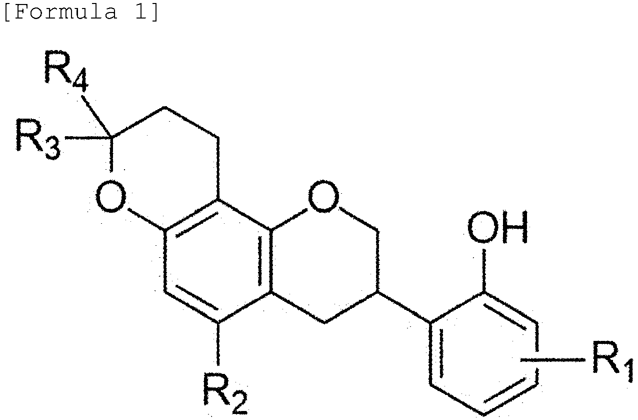 wherein
R₁ is a hydrogen atom, a substituted or unsubstituted linear or branched C₁-C₆ alkyl group, a halogen atom, a substituted or unsubstituted linear or branched C₁-C₆ alkoxy group, or a substituted or unsubstituted linear or branched C₁-C₄ thioalkyl group;
R₂ is a hydrogen atom, methyl, ethyl, methoxy or ethoxy; and
R₃ and R₄ are each independently a hydrogen atom or a C₁-C₂ alkyl group,
wherein the substituents of the substituted alkyl, the substituted alkoxy and the substituted thioalkyl are each independently a linear or branched C₁-C₅ alkyl group, a halogen atom, a linear or branched C₁-C₅ alkoxy group, or a linear or branched C₁-C₃ thioalkyl group.

2. The pharmaceutical composition of claim 1, wherein
R₁ is ethoxy, n-propoxy, isopropoxy, n-butoxy, 2-methylpropoxy, n-pentoxy, 2-methylbutoxy, 3-methylbutoxy, 2-ethylpropoxy, n-hexyloxy, 2-methylpentoxy, 3-methylpentoxy, 4-methylpentoxy, or 2-ethylbutoxy,
R₂ is a hydrogen atom, and
R₃ and R₄ are each independently methyl.

3. The pharmaceutical composition of claim 1, wherein the compound represented by Formula 1 comprises any one of the following compounds:

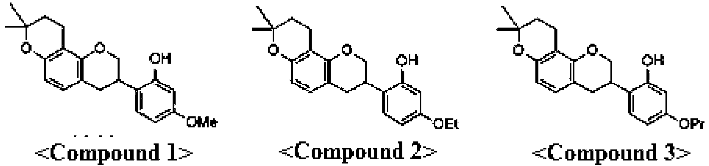

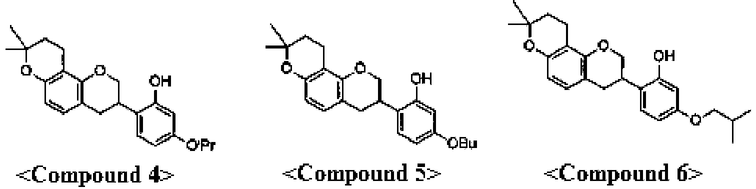

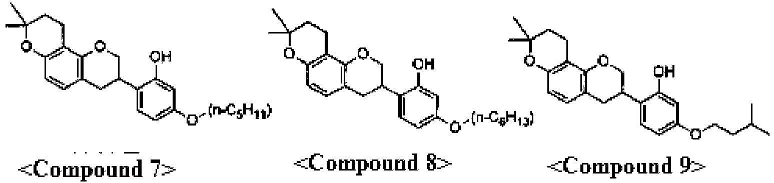

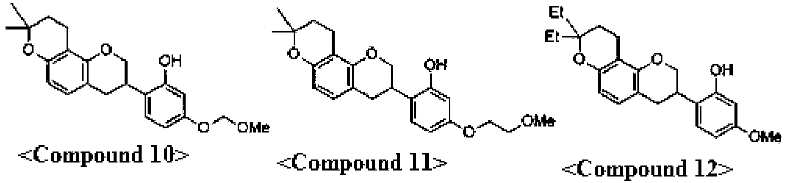

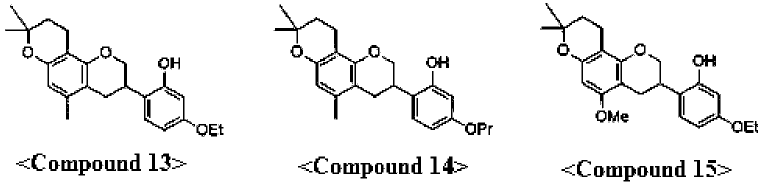

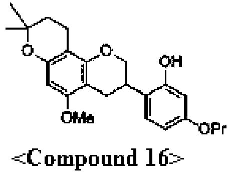

4. The pharmaceutical composition of claim 1, wherein the degenerative osteoarthritis is primary osteoarthritis or secondary osteoarthritis.

5. The pharmaceutical composition of claim 4, wherein the primary osteoarthritis is idiopathic osteoarthritis .

6. The pharmaceutical composition of claim 5, wherein the secondary osteoarthritis is chondromalacia patella or patellofemoral degenerative arthritis secondary to primary osteoarthritis.
